**Europäisches Patentamt**

**European Patent Office**

**Office européen des brevets**

(19)

(11) Veröffentlichungsnummer: **0 011 795**
**A1**

(12) # EUROPÄISCHE PATENTANMELDUNG

(21) Anmeldenummer: **79104570.1**

(22) Anmeldetag: **19.11.79**

(51) Int. Cl.³: **A 61 M 27/00**
**B 29 C 17/12, C 08 J 7/02**

(30) Priorität: **22.11.78 CH 11985/78**

(43) Veröffentlichungstag der Anmeldung:
**11.06.80 Patentblatt 80/12**

(84) Benannte Vertragsstaaten:
**AT BE CH DE FR GB IT LU NL SE**

(71) Anmelder: **Intermedicat GmbH**
**Gerliswilstrasse 45**
**CH-6020 Emmenbrücke(CH)**

(72) Erfinder: **Braun, Bernd, Dr.**
**Tränkelücke 1**
**Melsungen(DE)**

(74) Vertreter: **von Kreisler, Alek et al,**
**Deichmannhaus am Hauptbahnhof**
**D-5000 Köln 1(DE)**

(54) **Verfahren zur Herstellung von als Wunddrainagesonden dienenden Kunststoffschläuchen, insbesondere solcher mit antithrombogener Oberfläche, und hergestellte Kunststoffschläuche.**

(57) Zur Verwendung als Wunddrainagesonden bestimmte Kunststoffschläuche werden hergestellt, indem man einen mit durch Stanzen hergestellten Drainagelöchern (2) versehenen Kunststoffschlauch (1) zur Entfernung der beim Stanzen entstandenen, in das Lumen der Löcher und des Schlauches hineinragenden, Sekretverstopfungen und/oder Thrombozyten-Aggregationen verursachenden Unebenheiten (3) in ein organisches Lösungsmittel eintaucht, dort einige Minuten verweilen lässt und anschliessend das Lösungsmittel nach dem Herausnehmen des Schlauches sofort durch Trocknen entfernt.

Auf diese Weise werden Kunststoffschläuche erhalten, bei denen die Ränder der Stanzlöcher (2) glatt und frei von Unebenheiten, wie Fransen oder Fähnchen, sind, so dass es bei der Verwendung dieser Kunststoffschläuche als Wunddrainagesonden nicht zu unerwünschten, die Wundheilung beeinträchtigenden Sekretverstopfungen und/oder Thrombozyten-Aggregationen kommt.

Sollen die nach beschriebenen Verfahren hergestellten Schläuche noch mit einer antithrombogenen Oberfläche versehen werden, so unterwirft man sie einer Nachbehandlung mit einer 1-%igen Lösung von Polyäthylenacrylsäureester oder mit einem Gemisch von Tetrahydrofuran und Toluol, wodurch auf der Oberfläche der Schläuche eine dünne Schicht mit negativer Aufladung, welche eine Abstossung der Thrombozyten und deren Aggregaten bewirkt, erzeugt wird.

Fig. 1

./...

Fig. 2

Verfahren zur Herstellung von als Wunddrainagesonden dienenden Kunststoffschläuchen, insbesondere solchen mit antithrombogener Oberfläche

----------------------------------------------------------------

Um die Heilung von Wunden zu erleichtern oder zu verbessern, führt man in die geschlossene Wunde Drainageschläuche bzw. -sonden ein. Diese werden aus geeigneten, d.h. physiologisch unbedenklichen Kunststoffen, z.B. Polyvinylchlorid, Polyäthylen, Polyurethan, Silikonkautschuk oder Latexkautschuk hergestellt und mit Löchern versehen. Die Drainagewirkung dieser Schläuche bzw. Sonden kann noch verbessert werden, indem man sie mit ihrem, aus dem Körper herausragenden Ende an ein Vakuumsystem anschließt. Da die Gefahr besteht, daß die Schläuche beim Anlegen eines Vakuums zusammengezogen werden, ist es notwendig, Schläuche aus Materialien, die ine gewisse Shore-Härte aufweisen, zu verwenden, um diese Gefahr auszuschließen. Solche Materialien haben im allgemeinen eine minimale Shore-Härte von 6oA. Schläuche, die diesen Anforderungen genügen und eine ausreichende Shore-Härte aufweisen, sind die sog. Redondrainageschläuche (vgl. Langenbecks Arch.Chi.336, 163 - 172 (1974), Springer-Verlag 1974). Bei diesen Schläuchen ist jedoch das Ausstanzen der Drainagelöcher mit erheblichen SChwierigkeiten verbunden. Vor allem hat sich gezeigt,wie bei der Betrachtung mit der Lupe oder dem Mikroskop sichtbar wird, dass die so hergestellten Löcher nicht gleichmässig rund sind und keine glatten Ränder aufweisen. Vielmehr weisen sie zahlreiche Unebenheiten, insbesondere Fransen und feine Filmchen, auf, die sich sowohl auf dem oberen und dem inneren Rand als auch innerhalb der Wandstärke bilden. Diese Unebenheiten ragen in das Lumen des Loches und des Drainageschlauches hinein und verursachen so leicht Sekretverstopfungen und die Bildung von Thrombozyten-Aggregaten. Auf diese Weise wird häufig die Drainagewirkung auf ein ungenügendes Mass vermindert.

Ein weiterer Nachteil besteht darin, dass die Ränder der
Stanzlöcher nicht glatt sondern häufig scharfkantig sind,
wodurch eine Traumatisierung des Gewebes hervorgerufen
werden kann.

Wird nun ein derartiger Drainageschlauch in weiches Gewebe
eingebracht, wie dies z.B. bei Pankreas-, Gallen-, Bauch-
und Schädeloperationen der Fall ist, so kann es beim Anlegen eines Vakuums dazukommen, dass das weiche Gewebe durch
die Löcher eingesaugt und durch die vorhandenen scharfkantigen Ränder traumatisiert wird. Erfolgt diese Traumatisierung beispielsweise bei Darmgeweben, so können durch das,
in die Löcher eingesogene, traumatisierte Gewebe Bakterien
auswandern, was zu Peritonitis führen kann.

Ausgehend von dem im Vorhergehenden geschilderten Stand der
Technik war es Aufgabe der Erfindung, ein Verfahren zu entwickeln, welches es ermöglicht, für die Verwendung als
Wunddrainagesonden bestimmte Kunststoffschläuche herzustellen, die frei von den geschilderten Nachteilen sind und bei
deren Verwendung Sekretverstopfungen und die Bildung von
Thrombozyten-Aggregaten vermieden wird.

Die Lösung dieser Aufgabe gelingt mit Hilfe des erfindungsgemässen Verfahrens zur Herstellung von als Wunddrainagesonden dienenden Kunststoffschläuchen, das dadurch gekennzeichnet ist, dass man einen mit durch Stanzen hergestellten
Drainagelöchern versehenen Kunststoffschlauch zur Entfernung der beim Stanzen entstandenen, in das Lumen der Löcher
und des Schlauches hineinragenden, Sekretverstopfungen und/
oder Thrombozyten-Aggregationen verursachenden Unebenheiten
in ein organisches Lösungsmittel eintaucht, dort einige
Minuten verweilen lässt und anschliessend das Lösungsmittel nach dem Herausnehmen des Schlauches sofort durch Trocknen entfernt. Das Lösungsmittel löst die Unebenheiten
und löst das Material an, das die scharfkantigen Ränder
der Löcher bildet, wobei eine glatte Oberfläche um die
Löcher entsteht.

Bei einer bevorzugten Ausführungsform des beschriebenen Verfahrens verwendet man als organisches Lösungsmittel Toluol oder Tetrahydrofuran oder ein Gemisch
dieser Lösungsmittel. Andere Lösungsmittel, die verwendet werden können, sind Cyclohexanon, Methylenchlorid, Aceton, Xylol und ihre Gemische. Bevorzugt
wird ein 1 : 1-Gemisch von Toluol und Tetrahydrofuran.
Der Schlauch wird 1 bis 5 Minuten im Lösungsmittel
gelassen und dann herausgenommen. Im allgemeinen ist
eine Eintauchzeit von 2 bis 3 Minuten im Lösungsmittel
ausreichend. Man entfernt vorhandene Lösungsmittelreste durch Ausschleudern und streckt die Schläuche
durch Belasten mit einem Gewicht. Dann werden die
Schläuche in gestrecktem Zustand getrocknet, bis noch
vorhandene Lösungsmittelreste vollständig entfernt
sind. Das Trocknen erfolgt zweckmässiger Weise in
einem Vakuumtrockenschrank, ggfls. bei erhöhter Temperatur.

Der Schlauch ist vorzugsweise aus Polyvinylchlorid
oder Polyurethan hergestellt.

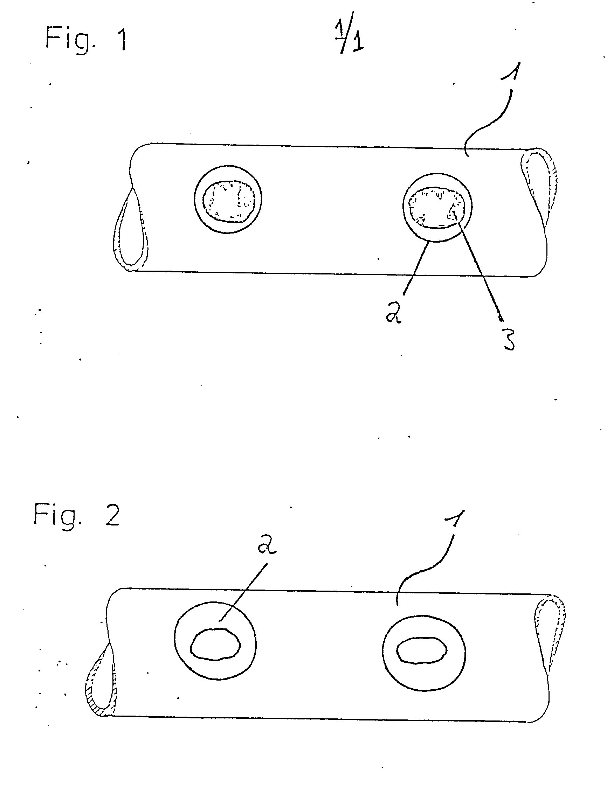

Auf die oben beschriebene Weise werden sämtliche an den
Stanzlöchern vorhandenen Unebenheiten entfernt. Dies
lässt sich durch einen Vergleich der Fig. 1 und 2 veranschaulichen.

Fig. 1 stellt einen Kunststoffschlauch 1 vor der Behandlung dar. Wie daraus ersichtlich, weisen die Stanzlöcher 2 zahlreiche Unebenheiten 3, die in das Lumen der Löcher und des Schlauches hineinragen, auf.

Fig. 2 zeigt den gleichen Schlauch 1 nach der Behandlung. Wie daraus ersichtlich, sind jetzt die Unebenheiten 3 gänzlich verschwunden und die Stanzlöcher 2 weisen glatte Ränder auf.

Häufig empfiehlt es sich, die so hergestellten Schläuche noch einer Nachbehandlung zu unterwerfen, durch welche der Schlauchoberfläche (innen und aussen) antithrombogene Eigenschaften verliehen werden. Dabei kann man wie folgt vorgehen: Man taucht einen nach dem vorstehend beschriebenen Verfahren hergestellten Kunststoffschlauch in eine reine 1%ige Lösung von Polyäthylenacrylsäureester, vorzugsweise ein niederer Alkylester wie z.B. Methylester oder Äthylester in einem organischen Lösungsmittel ein, entnimmt ihn nach kurzer Verweilzeit aus der Tauchlösung und trocknet ihn. Auf diese Weise wird auf der Oberfläche des Schlauches eine dünne Schicht mit negativer Aufladung, welche eine Abstossung der Thrombozyten und deren Aggregaten bewirkt, erzeugt. Das Lösungsmittel für den Polyäthylenacrylsäureester kann Tetrahydrofuran allein oder vorzugsweise ein Gemisch mit Toluol sein, wie z.B. ein 1 : 1-Gemisch.

Der für das Aufbringen der dünnen Schicht verwendete Polyäthylenacrylsäureester enthält vorteilhaft ein Verhältnis von 95 : 5 bis 7o : 3o und vorzugsweise ein Verhältnis von 8o : 2o von Polyäthylen zu Acrylsäure.

Um die aufgebrachte Polyäthylenacrylsäureester-Schicht zu glätten, kann der Schlauch erneut in eine Lösung mit sehr geringer Konzentration wie z.B. eine o,5 %ige Lösung von Polyäthylenacrylsäureester in einem Lösungsmittel wie z.B. ein Gemisch (1 : 1) von Toluol und Tetrahydrofuran eingetaucht werden. Alternativ kann die erste und einzige Schicht des Polyäthylenacrylsäureesters dadurch geglättet werden, daßman den Schlauch wiederum in Tetrahydrofuran oder ein Gemisch von Toluol und Tetrahydrofuran, vorteilhaft im Verhältnis von 1 : 1, eintaucht, das keinen Polyäthylenacrylsäureester enthält.

Durch die beschriebene Behandlung wird vermieden, daß es im Fall von Nachblutungen zur Aggregation von Thrombozyten an der Schlauchoberfläche und damit zur Thrombenbildung kommt.

-6-

Patentansprüche
------------------------

1. Verfahren zur Herstellung von als Wunddrainagesonden dienenden Kunststoffschläuchen, dadurch gekennzeichnet, dass
man einen mit durch Stanzen hergestellten Drainagelöchern
versehenen Kunststoffschlauch zur Entfernung der beim
Stanzen entstandenen, in das Lumen der Löcher und des
Schlauches hineinragenden, Sekretverstopfungen und/oder
Thrombozyten-Aggregationen verursachenden Unebenheiten in
ein organisches Lösungsmittel eintaucht, dort einige Minuten verweilen lässt und anschliessend das Lösungsmittel
nach dem Herausnehmen des Schlauches sofort durch Trocknen entfernt.

2. Verfahren nach Patentanspruch 1, dadurch gekennzeichnet,
dass man als organisches Lösungsmittel Toluol, Tetrahydrofuran oder ein Gemisch davon verwendet.

3. Verfahren nach Anspruch 1, dadurch gekennzeichnet, daß
man den Kunststoffschlauch 1 bis 5 Minuten, vorzugsweise
2 bis 3 Minuten in dem organischen Lösungsmittel verweilen lässt.

4. Verfahren nach Anspruch 1, dadurch gekennzeichnet, daß
man das organische Lösungsmittel durch Trocknen im Vakuum
entfernt.

5. Verfahren nach Anspruch 1, dadurch gekennzeichnet, daß
man einen Schlauch, der von Unebenheiten um die Löcher
befreit ist, in eine reine 1 %ige Lösung von Polyäthylenacrylsäureester in einem organischen Lösungsmittel eintaucht, nach kurzer Verweilzeit aus der Tauchlösung entfernt und trocknet, wodurch auf der Oberfläche des Schlauches eine dünne Schicht mit negativer Aufladung, welche
eine Abstossung der Thrombozyten und deren Aggregaten bewirkt, erzeugt wird.

6. Verfahren nach Anspruch 5, dadurch gekennzeichnet, daß
   der Schlauch weiter geglättet wird, indem man den Schlauch
   erneut für eine kurze Zeit in eine Lösung eines Polyäthylenacrylsäureesters in einem organischen Lösungsmittel taucht,
   den Schlauch aus der Lösung entfernt und trocknet.

7. Verfahren nach Anspruch 5, dadurch gekennzeichnet, daß
   der Schlauch weiter geglättet wird, indem man ihn in
   Tetrahydrofuran allein oder in ein Gemisch von Tetrahydrofuran und Toluol eintaucht.

8. Kunststoffschlauch, hergestellt nach Ansprüchen 1 - 7.

Fig. 1  ¹⁄₁

Fig. 2

| | | | |
|---|---|---|---|
| **Europäisches Patentamt** | **EUROPÄISCHER RECHERCHENBERICHT** | Nummer der Anmeldung **0011795** | |
| | | EP 79 10 4570 | |

| **EINSCHLÄGIGE DOKUMENTE** | | | **KLASSIFIKATION DER ANMELDUNG (Int.Cl. 3)** |
|---|---|---|---|
| Kategorie | Kennzeichnung des Dokuments mit Angabe, soweit erforderlich, der maßgeblichen Teile | betrifft Anspruch | |
| | US - A - 2 972 779 (COWLEY) <br> * Spalte 1, Zeilen 67-73; Patentansprüche * <br><br> -- | 1,2,8 | A 61 M 27/00 <br> B 29 C 17/12 <br> C 08 J 7/02 |
| | US - A - 3 524 447 (EVANS) <br> * Spalte 2, Zeilen 12-24 * <br><br> -- | 1,2,7, 8 | |
| | US - A - 3 446 890 (EMERY) <br> * Spalte 2, Zeilen 31-44; Spalte 4, Zeile 58 * <br><br> -- | 1,3 | **RECHERCHIERTE SACHGEBIETE (Int. Cl. 3)** |
| | US - A - 3 680 568 (JAMES) <br> * Zusammenfassung * <br><br> -- | | A 61 M <br> C 08 J <br> B 29 C <br> B 26 F |
| | FR - A - 2 045 472 (IMAGINEERING) <br> * Seite 4, Zeile 33 - Seite 5, Zeile 24; Patentanspruch 5 * <br><br> -- | 1,2,3 | |
| | FR - A - 2 311 559 (MEADOX) <br> * Patentanspruch 1 * <br><br> -- | 5-8 | **KATEGORIE DER GENANNTEN DOKUMENTE** <br> X: von besonderer Bedeutung <br> A: technologischer Hintergrund <br> O: nichtschriftliche Offenbarung <br> P: Zwischenliteratur |
| | FR - A - 2 225 178 (MEADOX) <br> * Patentanspruch 1 * <br><br> -- | 5-8 | T: der Erfindung zugrunde liegende Theorien oder Grundsätze <br> E: kollidierende Anmeldung <br> D: in der Anmeldung angeführtes Dokument |
| | DE - A - 2 622 502 (REHAU PLASTICS) <br> * Seite 8, Zeilen 8-10 * | 5-8 | L: aus andern Gründen angeführtes Dokument <br> &: Mitglied der gleichen Patentfamilie, übereinstimmendes Dokument |

---

Der vorliegende Recherchenbericht wurde für alle Patentansprüche erstellt.

| Recherchenort | Abschlußdatum der Recherche | Prüfer |
|---|---|---|
| Den Haag | 28-02-1980 | CORDENIER |

EPA form 1503.1   06.78